# EUROPEAN PATENT SPECIFICATION

(11) **EP 1 852 512 B1**
(45) Date of publication and mention of the grant of the patent: **02.11.2011**
(21) Application number: 06290698.7
(22) Date of filing: 02.05.2006
(51) Int. Cl.: C12Q 1/68, C07H 21/00

(54) **Microorganisms detection and enumeration method**
Methode zur Bestimmung und Auszählung von Mikroorganismen
Méthode pour la détection et l'énumération de microorganismes

(43) Date of publication of application: 07.11.2007
(73) Proprietor: UNIVERSITE PIERRE ET MARIE CURIE, 75005 Paris (FR); Centre National de la Recherche Scientifique (CNRS), 75794 Paris Cedex 16 (FR)
(72) Inventor: Baudart, Julia, 66650 Banyuls sur mer (FR); Lebaron, Philippe, 66650 Banyuls sur mer (FR)
(74) Representative: Cabinet Plasseraud

(56) References cited:
- WO-A-89/04876
- KOGURE K. ET AL.: "A TENTATIVE DIRECT MICROSCOPIC METHOD FOR COUNTING LIVING MARINE BACTERIA" CANADIAN JOURNAL OF MICROBIOLOGY, vol. 25, no. 3, 1979, pages 415-420, XP009070718 ISSN: 0008-4166
- FUCHS B.M. ET AL.: "Unlabeled helper oligonucleotides increase the in situ accessibility to 16S rRNA of fluorescently labeled oligonucleotide probes" APPLIED AND ENVIRONMENTAL MICROBIOLOGY, WASHINGTON,DC, US, vol. 66, no. 8, August 2000 (2000-08), pages 3603-3607, XP002266643 ISSN: 0099-2240
- FUCHS B.M. ET AL.: "Flow cytometric analysis of the in situ accessibility of Escherichia coli 16S rRNA for fluorescently labeled oligonucleotide probes" APPLIED AND ENVIRONMENTAL MICROBIOLOGY, vol. 64, no. 12, December 1998 (1998-12), pages 4973-4982, XP002393711 ISSN: 0099-2240
- YILMAZ L S. ET AL.: "Making all parts of the 16S rRNA of Escherichia coli accessible in situ to single DNA oligonucleotides" APPLIED AND ENVIRONMENTAL MICROBIOLOGY, vol. 72, no. 1, January 2006 (2006-01), pages 733-744, XP002393712 ISSN: 0099-2240
- BAUDART J ET AL: "Rapid and sensitive enumeration of viable diluted cells of members of the family Enterobacteriaceae in freshwater and drinking water", APPLIED AND ENVIRONMENTAL MICROBIOLOGY, AMERICAN SOCIETY FOR MICROBIOLOGY, US LNKD- DOI:10.1128/AEM.68.10.5057-5063.2002, vol. 68, no. 10, 1 October 2002 (2002-10-01), pages 5057-5063, XP002273051, ISSN: 0099-2240
- KALMBACH SIBYLLE ET AL: "Dynamics of biofilm formation in drinking water: Phylogenetic affiliation and metabolic potential of single cells assessed by formazan reduction and in situ hybridization", FEMS MICROBIOLOGY ECOLOGY, vol. 22, no. 4, 1997, pages 265-279, ISSN: 0168-6496
- NISHIMURA MASAHIKO ET AL: "A new method to detect viable bacteria in natural seawater using 16S rRNA oligonucleotide probe", JOURNAL OF OCEANOGRAPHY, vol. 49, no. 1, 1993, pages 51-56, XP009133885, ISSN: 0916-8370
- MCGREGOR, D.P., ET AL.: "SIMULTANEOUS DETECTION OF MICROORGANISMS IN SOIL SUSPENSION BASED ON PCR AMPLIFICATION OF BACTERIAL 16S RRNA FRAGMENTS", 19960101, vol. 21, no. 3, 1 January 1996 (1996-01-01), XP001247274, ISSN: 0736-6205
- REGNAULT BEATRICE ET AL: "Oligonucleotide probe for the visualization of Escherichia coli/Escherichia fergusonii cells by in situ hybridization: Specificity and potential applications", RESEARCH IN MICROBIOLOGY, vol. 151, no. 7, September 2000 (2000-09), pages 521-533, ISSN: 0923-2508

### SEQUENCE LISTING

<110> université Pierre et Marie Curie BAUDART, Julia LEBARON, Philippe
<120> MICROORGANISMS DETECTION AND ENUMERATION METHOD
<130> BFF060198
<160> 36
<170> PatentIn version 3.3
<210> 1
   <211> 23
   <212> DNA
   <213> Escherichia coli
<400> 1
   gcaaaggtat taactttact ccc 23
<210> 2
   <211> 24
   <212> DNA
   <213> Escherichia coli
<400> 2
   gagactcaag attgccagta tcag 24
<210> 3
   <211> 30
   <212> DNA
   <213> Escherichia coli
<400> 3
   ttcctccccg ctgaaagtac tttacacccg 30
<210> 4
   <211> 30
   <212> DNA
   <213> Escherichia coli
<400> 4
   cggtgcttct tctgcgggta acgtcaatga 30
<210> 5
   <211> 30
   <212> DNA
   <213> Escherichia coli
<400> 5
   atgcagttcc caggttgagc ccggggattt 30
<210> 6
   <211> 30
   <212> DNA
   <213> Escherichia coli
<400> 6
   cgctacacct ggaattctac ccccctctac 30
<210> 7
   <211> 18
   <212> DNA
   <213> Legionella spp
<400> 7
   ctggtgttcc ttccgatc 18
<210> 8
   <211> 18
   <212> DNA
   <213> Legionella spp
<400> 8
   tcggacgcag gctaatct 18
<210> 9
   <211> 19
   <212> DNA
   <213> Legionella spp
<400> 9
   cctcctcccc actgaaagt 19
<210> 10
   <211> 20
   <212> DNA
   <213> Legionella spp
<400> 10
   cactgtatgt caagggtagg 20
<210> 11
   <211> 18
   <212> DNA
   <213> Legionella spp
<400> 11
   aaggcatatt cctacgcg 18
<210> 12
   <211> 30
   <212> DNA
   <213> Legionella spp
<400> 12
   tctacgcatt tcaccgctac accggaaatt 30
<210> 13
   <211> 30
   <212> DNA
   <213> Legionella spp
<400> 13
   cagtattagg ccaggtagcc gccttcgcca 30
<210> 14
   <211> 30
   <212> DNA
   <213> Legionella spp
<400> 14
   taaagcgcca ggccttacgg tccccggctt 30
<210> 15
   <211> 30
   <212> DNA
   <213> Legionella spp
<400> 15
   tggtaggcct ttaccctacc aactagctaa 30
<210> 16
   <211> 30
   <212> DNA
   <213> Legionella spp
<400> 16
   gctttacaac cctcaggcct tcttcacaca 30
<210> 17
   <211> 30
   <212> DNA
   <213> Legionella spp
<400> 17
   gtccaattat ctagctctta acctaataat 30
<210> 18
   <211> 30
   <212> DNA
   <213> Legionella spp
<400> 18
   taaggttctt cgcgttgcat cgaattaaac 30
<210> 19
   <211> 30
   <212> DNA
   <213> Legionella spp
<400> 19
   ccgaaggcag gaatgcatct ctgcaaactt 30
<210> 20
   <211> 30
   <212> DNA
   <213> Legionella spp
<400> 20
   ttactcaccc gttcgccact cgccatccat 30
<210> 21
   <211> 30
   <212> DNA
   <213> Legionella spp
<400> 21
   cttgagtttc cccaagttgt ccccctcttt 30
<210> 22
   <211> 18
   <212> DNA
   <213> Legionella pneumophila
<400> 22
   atctgaccgt cccaggtt 18
<210> 23
   <211> 18
   <212> DNA
   <213> Legionella pneumophila
<400> 23
   agctttcatc caaagata 18
<210> 24
   <211> 30
   <212> DNA
   <213> Legionella pneumophila
<400> 24
   aagcccagga atttcacaga taacttaatc 30
<210> 25
   <211> 30
   <212> DNA
   <213> Legionella pneumophila
<400> 25
   ccctctccca tactcgagtc aaccagtatt 30
<210> 26
   <211> 30
   <212> DNA
   <213> Legionella pneumophila
<400> 26
   ttatgcggta ttagcttgag tttccccaag 30
<210> 27
   <211> 30
   <212> DNA
   <213> Legionella pneumophila
<400> 27
   aatcttaaag cgccaggccc gaaggtcccc 30
<210> 28
   <211> 20.
   <212> DNA
   <213> Salmonella spp
<400> 28
   acagcacatg cgcttttgtg 20
<210> 29
   <211> 18
   <212> DNA
   <213> Salmonella spp
<400> 29
   aatcacttca cctacgtg 18
<210> 30
   <211> 18 <212> DNA
   <213> Salmonella spp
<400> 30
   gcagtcacac aggtaaac 18
<210> 31
   <211> 30
   <212> DNA
   <213> salmonella spp
<400> 31
   tacggggctg tcaccctgta tcgcgcgcct 30
<210> 32
   <211> 30
   <212> DNA
   <213> Salmonella spp
<400> 32
   accacgtgtc ccgccctact catcgagctc 30
<210> 33
   <211> 30
   <212> DNA
   <213> Salmonella spp
<400> 33
   tcagcgtgcc ttctcccgaa gttacggcac 30
<210> 34
   <211> 30
   <212> DNA
   <213> salmonella spp
<400> 34
   tatcttcgac tgacttcagc tccatgagta 30
<210> 35
   <211> 30
   <212> DNA
   <213> Salmonella spp
<400> 35
   ctgtgctccc actgcttgta cgtacacggt 30
<210> 36
   <211> 31
   <212> DNA
   <213> Salmonella spp
<400> 36
   taagtcgctg accccattat acaaaaggta c 31

## Description

The present invention concerns the domain of health hazards associated to the presence of pathogens in an environment. The present invention relates to methods for detecting and enumerating highly-diluted viable microorganisms in a sample belonging to the genomic species *Escherichia coli* , and to associated kits. The present invention further relates to helper probes for binding specifically to ribosomal RNA of bacteria *Escherichia coli.*

Water managment is one of the most important issue of this century. The contamination diagnosis of an environment is essential to evaluate the health risk associated to the presence of pathogens in said environment. Pathogens which are associated to health hazard are for example *E*. *Coli, Salmonella* or *Legionella.*

*E. coli* is a commensal bacterium of the colon of man and of warm-blooded animals. For this reason, its presence in a sample of water, food or from the environment, is interpreted as an indication of fecal contamination (indicative bacterium). Strains of the genomic species of *E. coli* can be pathogenic: they can cause different infections in man or in animals (urinary infections, choleriform or hemorrhagic diarrhea, dysentery syndrome, hemolytic and uremic syndrome, septicemia, neonatal meningitis, various purulent infections). The enumeration of *E. coli* is thus essential in order to estimate the hygienic quality of water or food.

*Legionella* are ubiquitous inhabitants of wet soil as well as non-marine aquatic habitats. Ideal conditions for their propagation are temperatures between 25°C and 55°C. Consequently, they can also be found in habitats created by humans, such as warm and cold water installations, cooling towers of air conditioning systems and water humidifiers. As intracellular parasites of amoebae and ciliates, they can also survive unfavorable living conditions, such as extreme temperatures and chlorination of water. *Legionella,* in particular *Legionella pneumophilia,* are pathogens: in human, they cause an acute bacterial pneumonia with facultative lethal course, generally known as "legionnaire's disease".

*Salmonella* spp. are ubiquitous enteric bacteria. They are the etiologic agents of food-borne salmonellosis and also the agents that cause thyphoid and parathyphoid fevers. Although food products, including shellfish, are the most common sources of salmonellosis, *Salmonella* is a prime example of a water transmitted pathogen. *Salmonella* are often detected in sewage, freshwater, marine coastal water and ground water. *Salmonella* spp. can survive for long periods in natural waters, thus the persistence of specific and epidemic strains is of great concern in public health.

Therefore, the contamination of water, food or environment by microorganisms, especially bacteria, is of great concern in public health; and rapid methods for detecting and enumerating these pathogens are critical to evaluate the health risk associated to the presence of these pathogens.

Traditional detection and enumeration of microorganisms pathogens are realized by means of cell culturing. However these cell culture methods are of limited interest: they are fastidious, in particular because the pathogens in the sample are generally highly diluted, and the culture medium may have the undesired side effect of partially inhibiting the growth of the pathogens, thus leading to an underestimation of the pathogens. Thus, these methods are not satisfactory because they are time consuming and do not allow a reliable enumeration of pathogens.

Methods based on the detection of specific nucleotide sequences of the microorganisms have been developped, such as the one based on PCR or RT-PCR techniques (Yamamoto *et al.* 1993, Bej *et al.* 1991, Devos *et al.* 2005). However, these PCR-based methods, when used in the field of environmental microbiology, do not give the expected qualitative results because of polymerisation reaction inhibitors present in the samples; in addition, these methods require a prior DNA purification step, leading to DNA loss and consequently to an underestimation of pathogens. As PCR based methods are considered to be qualitative but not quantitative, a real-time PCR based method was designed (Yanez *et al.* 2005). However, this newly designed real-time PCR based method did not allow discriminating between viable and non viable cells, and lead to an overestimation of the pathogens number and thus to wrong results. Thus the PCR based methods do not allow an accurate enumeration of viable microorganism in a sample.

*In situ* hybridization is an interesting alternative in gene amplification. The method using this technic is called fluorescent *in situ* hybridisation (FISH). An oligonucleotidic probe labeled by a fluorescent substance penetrates into bacteria cells, previously treated to facilitate this step. If the ribosomal nucleic acids (rRNA) have a complementary (target) sequence to the probe, the probe will fix to its target and will not be removed by washing. The bacteria having retained the probe will then emit a fluorescent signal, that can be quantifyied by epifluorescent microscopy, flow cytometry or solid phase cytometry. rRNA form the preferred target in FISH because of their highly conserved regions and their high number of copies per cell. Examples of microorganisms detection methods using rRNA hybridisation techniques can be found in US patents 5,288,611 and US 5,426,025. A real advantage of the FISH method is that it allows direct detecting of bacteria within hours, instead of the days required with classical culture-based methods. However, this conventional FISH method (using monolabeled probes) seemed to be unsuitable to the detection of highly diluted microorganisms in a sample because the amount of ribosomic material was very limited, thus leading to a weak and undetectable flurorescent signal. In addition, the FISH method alone does not allow discriminating between viable and non viable cells, leading to an overestimation of the pathogens number.

These drawbacks have been overcome by combining the FISH method with a method allowing the bacterial cells viability control, called the Direct Viable Counts test (DVC) (Kogure *et al.* 1987). However, the combination of the DVC and the FISH standard techniques are not sufficient to obtain a detectable fluorescent signal when combining with cells detection systems used for rare events detection (Solid Phase cytometer for example).

Recently, Baudart *et al.* assessed the specificity and sensitivity of a DVC-FISH method combined with a fluorescence amplification technique based on tyramide signal amplification (TSA), using a solid phase cytometer (Baudart *et al.* 2002). In this study, a hybridization protocol was developed, in which the probe was used in combination with HRP and was revealed by the TSA system, with the aim of increasing the fluorescence intensity of the hybridized cells. This FISH technique is also commonly called CARD-FISH (CAtalyzed Reporter Deposit-FISH). The results show that this method allowed the detection of one targeted cell in approximately 10⁸ non targeted cells. However, a drawback of many signal amplification systems such as the HRP-probe-TSA system is that they require a diffusion of large-molecular-weight molecule such as enzymes, antibodies, or (strept)avidin within target cells. The diffusion of these large-molecular-weight molecule requires prealable enzymatique permeabilization of cells, which can be strains or species dependent (Amann *et al*., 1995). In such an extent that it may present a risk of loss of target molecules or complete cell lysis. Thus, enumeration of microorganism in a sample using such signal amplification system may be erroneous, and requires different time-consuming steps (paraformaldehyde fixation, permeabilization and final labelling of the HRP activity). Consequently, because this procedure includes to much steps, it is not appropriate for a routine application in quality control laboratories controls.There is thus still a need for methods with an improved liability, which allow quick and ultra-sensitive detection and enumeration of viable and highly-diluted pathogen cells, are capable of strongly amplifying the fluorescence intensity of the hybridized pathogen cells, and avoid any loss or lyses of said pathogen cells before the enumeration has occurred.

Thus the present invention relates to an ultra-sensitive and rapid method of detection and enumeration of viable microorganisms using combined FISH and DVC methods, and having an improved alternative system to amplify the fluorescence intensity of the hybridized cells.

This invention also relates to said improved system, including using helper oligonucleotidic probes, functional to amplify the fluorescence intensity of the hybridized cells.

As *Escherichia coli, Legionella* spp., *Legionella pneumophila* and *Salmonella* spp. are nowdays major pathogens whose presence is assessed to determine the quality of water, food or of an environment, the Applicant designated helper probes to allow the detection and enumeration of such highly-diluted viable cells to examplify their invention.

One skilled in the art would easily deduce from the present invention how to detect and enumerate cells of other species.

According to the invention, the method for detecting and enumarating viable Escherichia coli microorganisms in a sample suspected of containing said microorganisms comprises:
(1) contacting said sample with a cell nutritive resource and a cellular proliferation inhibitor,
(2) contacting said sample with at least one fluorescence labeled oligonucleotidic probe able to specifically hybridize at least one portion of ribosomal nucleic acids of said *Escherichia coli* microorganisms, said probe being chosen among ECOLI probe (SEQ ID n°1) or Colinsitu probe (SEQ ID n°2) and all sequences having 70%, preferably 80% and more preferably 90% of identity with SEQ ID n°1 or SEQ ID n°2, thereby performing hybridization,
(3) contacting said sample, with a least one helper probe able to hybridize at least one portion of said ribosomal nucleic acids of said *Escherichia coli* microorganisms,
(4) detecting and quantifying the fluorescent signal of said fluorescence labeled oligonucleotide probe, thereby detecting and enumerating the number of viable *Escherichia coli* microorganisms in said sample.

Step (1) of the above method allows the measurement at single cell level of the cellular viability, represented by metabolic synthesis activity. The cellular viability was measured by the modified DVC procedure of Kogure et al. (Kogure *et al*., 1987). This assay includes a bacterial cell metabolism revivification step in the presence of a DNA gyrase inhibitor, such as nalidixic acid, which stops cell division, increases the intracellular rRNA content and the cellular length of sensitive cells.

Step (2) of the above method comprises a standard FISH procedure. Prior to hybridization, the DVC treated cells are preferably fixed and permeabilized. Fixing and permeabilizing a microorganism includes treating the external membrane or envelop to be permeable for oligonucleotidic probes. For fixing, a low percentage of paraformaldehyde solution is usually used. Others well-known methods include for example ethanol, methanol, diluted formaldehyde solution, enzymatic treatments or the like.
Hybridization is then performed in an hybridization buffer containing at least one fluorescence labeled oligonuleotidic probe. These oligonucleotidic probes, which comprise an oligonucleotide and a marker linked thereto, can then penetrate the external membrane or envelop and bind to the target sequence corresponding to the oligonucleotidic probe in the cell. Binding is to be understood as formation of hydrogen bonds between complementary nucleic acid pieces. In the FISH technique, these probes are complementary to a certain region on the ribosomal target sequence. They are generally small, 15-30 bases long, single-stranded deoxyribonucleic acid pieces and are directed against a target region which is typical for a microorganism species, species, genus, group or phylogenetic family.

Step (3) of the above method aims to amplify the fluorescent signal resulting from the hybridization of the labeled probes with their ribosomal target sequence. The helper probes according to the invention comprise oligonucleotidic probes that bind sequences adjacent to the labeled probe target sequences, in order to increase the *in situ* accessibility of said labeled probes and consequently to increase the fluorescent signal of said labeled probes.
These helper probes preferably are non-labeled probes and are specific (but not exclusively) of the targeted microorganism. They are preferably small, single-stranded deoxyribonucleic acid pieces, and may comprise 10 to 50 nucleotides, preferably 12 to 40 nucleotides and more preferably 14 to 25 nucleotides. In the meaning of this invention, a sequence adjacent to a labeled probe target sequence figures a target sequence which is in a region of 100 bp, preferably 75 bp, and more preferably 30 bp on either side of the labeled probe target sequence. In a prefered embodiment of the invention, helper probes are 70%, preferably 80%, more preferably 90%, and most preferably 100% complementary of their target sequence.

According to a preferred embodiment of the invention, Step (2) and (3) are carried out simultaneously.

Step (4) of the above method comprises the detection and the quantification of the fluorescent signal of said labeled probes by a solid phase cytometer or any equivalent detection instrument.

In this invention, the following term are defined as follows:
"to detect" a microorganism in a sample means to perceive the presence of a microorganism in a sample;
"to enumerate" microorganisms in a sample means to count the microorganisms present in a sample;
a "sample" may mean a sample of water as sewage water, freshwater, marine coastal water, ground water for example, a sample of liquid biological material as blood or urine samples, or a sample of food, or any type of sample suitable for use in the detection device;
"microorganisms" means any viable organism of microscopic size, including bacteria, cyanobacteria, chlamydiae, fungi, algae, protozoa and viruses;
"highly diluted" microorganisms means microorganisms in low concentration or in limited number, for example in an amount less than 100 in 1 ml.

In a preferred embodiment of the invention, viable microorganisms that can be detected and enumerated according to the above method are *Escherichia coli, Legionella spp, Legionella pneumophila* and *Salmonella spp.*

In a preferred embodiment of the invention, the above method allows the detection and enumeration of *Escherichia coli.* In this embodiment, the preferred specific labeled oligonucleotidic probes for use in step (2) are:
- the ECOLI probe (SEQ ID n°1) described in Mc Gregor *et al.* (Mc Gregor *et al.,* 1996),
- the Colinsitu probe (SEQ ID n°2) described in Regnault *et al.* (Regnault *et al.,* 2000),
- and all sequences having 70%, preferably 80% and more preferably 90% of identity with SEQ ID n°1 or SEQ ID n°2;

In this embodiment, the preferred helper probes for use in step (3) are:
- the HECOLIL probe (SEQ ID n°3),
- the HECOLIR probe (SEQ ID n°4),
- the HColinsituL probe (SEQ ID n°5),
- the HColinsituR probe (SEQ ID n°6).
- and all sequences having 70%, preferably 80% and more preferably 90% of identity with SEQ ID n°3, SEQ ID n°4, SEQ ID n°5 or SEQ ID n°6; in this embodiment of the invention, the ECOLI probe is preferably used in combination with HECOLIL and HECOLIR probes, and the Colinsitu probe is preferably used in combination with HColinsituL and HColinsituR probes.

| Probe | Sequence 5'-3' | rRNA target position | Tm/%GC/n | Specificity |
|---|---|---|---|---|
| ECOLI | GCAAA GGTAT TAACT TTACT CCC | 16S, 453-475 | 49/39/23 | *E. coli*/ *Shigella spp* |
| HECOLIL | TTCCT CCCCG CTGAA AGTAC TTTAC ACCCG | 16S, 423-452 | | |
| HECOLIR | CGGTG CTTCT TCTGC GGGTA ACGTC AATGA | 16S, 476-505 | | |
| Colinsitu | GAGAC TCAAG ATTGC CAGTA TCAG | 16S, 637-660 | 52/96/29 | *E. coli*/ *Shigella spp* |
| HColinsituL | ATGCA GTTCC CAGGT TGAGC CCGGG GATTT | 16S, 607-636 | | |
| HColinsituR | CGCTA CACCT GGAAT TCTAC CCCCC TCTAC | 16S, 661-690 | | |

Furthermore described herein is
the detection and enumeration of *Legionella* spp. In this embodiment, the preferred specific labeled oligonucleotidic probes for use in step (2) of the method are:
- the LEG705 probe (SEQ ID n°7) described in Manz *et al.* (Manz *et al.* 1995),
- the LEG226 probe (SEQ ID n°8) described in Manz *et al.* (Manz *et al.* 1995),
- the Legall11 probe (SEQ ID n°9) described in Leskela *et al.* (Leskela *et al.* 2005),
- the Legall22 probe (SEQ ID n°10) described in Leskela *et al.* (Leskela *et al.* 2005),
- the Leg120v probe (SEQ ID n°11) described in Buchbinder *et al.* (Bushbinder *et al.* 2002)
- and all sequences having 70%, preferably 80% and more preferably 90% of identity with SEQ ID n°7, SEQ ID n°8, SEQ ID n°9, SEQ ID n°10 or SEQ ID n°11;

In this embodiment, the preferred helper probes for use in step (3) of the method are:
- the HLEG705L probe (SEQ ID n°12),
- the HLEG705R probe (SEQ ID n°13),
- the HLEG226L probe (SEQ ID n°14),
- the HLEG226R probe (SEQ ID n°15),
- the HLegall11L probe (SEQ ID n°16),
- the HLegall11R probe (SEQ ID n°17),
- the HLegall22L probe (SEQ ID n°18),
- the HLegall22R probe (SEQ ID n°19),
- the HLeg120vL (SEQ ID n°20),
- the HLeg120vR (SEQ ID n°21)
- and all sequences having 70%, preferably 80% and more preferably 90% of identity with SEQ ID n°12, SEQ ID n°13, SEQ ID n°14, SEQ ID n°15, SEQ ID n°16, SEQ ID n°17, SEQ ID n°18, SEQ ID n°19, SEQ ID n°20 or SEQ ID n°21;
in this embodiment , LEG705 probe is preferably used in combination with HLEG705L and HLEG705R probes, the LEG226 probe is preferably used in combination with HLEG226L and HLEG226R probes, the Legall11 probe is preferably used in combination with HLegall11L and HLegall11R probes, the Legall22 probe is preferably used in combination with HLegall22L and HLegall22R probes, and the Leg120v probe is preferably used in combination with HLeg120vL and HLeg120vR probes.

| Probe | Sequence 5'-3' | rRNA target position | Tm/%GC/n | Specificity |
|---|---|---|---|---|
| **LEG705** | CTGGT GTTCC TRCCG ATC | 16S, 705-722 | 47/56/18 | *Legionella* spp. |
| **HLEG705L** | TcTAC GCATT TCACC GCTAC ACCGG AAATT | 16S, 675-704 | | |
| **HLEG705R** | CAGTA TTAGG CCAGG TAGCC GCCTT CGCCA | 16S, 721-752 | | |
| **LEG226** | TCGGA CGCAG GCTAA TCT | 16S, 226-243 | 51/56/18 | *Legionella* spp. |
| **HLEG2206L** | TAAAG CGCCA GGCCt tACGG TCCCC GGCTT | 16S, 196-225 | | |
| **HLEG226R** | TGGTA GGCCt TTACC CTACC AACTA GCTAA | 16S, 244-273 | | |
| **Legall11** | CCTCC TCCCC ACTGA AAGT | 16S, 435-454 | 50/18/19 | *Legionella* spp. |
| **HLegall11L** | GCTTT ACAAC CCTCA GGCCT TCTTC ACACA | 16S, 405-434 | | |
| **HLegall11R** | GTCCA aTTAT CTAGC TCTTA ACCTA ATAAT | 16S, 455-484 | | |
| **Legall22** | CACTG TATGT CAAGG GTAGG | 16S, 984-1033 | 50/58/19 | *Legionella* spp*.* |
| **HLegall22L** | TAAGG TTCTT CGCGT TGCAT CGAAT TAAAC | 16S, 954-983 | | |
| **HLega1122R** | CCGAA GGCAG GAATG CATCT CTGCA AACTT | 16S, 1004- 1033 | | |
| **Leg120v** | AAGGCATATTCCTACGCG | 16S, 121-139 | 48/50/18 | *Legionella* spp. |
| **HLeg120vL** | TTACT CACCC GTTCG CCACT CGCCA TCCAT | 16S, 120-91 | | |
| **HLeg120vR** | CTTGA GTTTC CCCAA GTTGT CCCCC TCTTt | 16S, 140-169 | | |

Furthermore described herein is the detection and enumeration of *Legionella pneumophila.* In this embodiment, the preferred specific labeled oligonucleotidic probes for use in step (2) of the method are:
- the LEGPNE1 probe (SEQ ID n°22) described in Grimm *et al.* and Declerck *et al.* (Grimm *et al.* 1998 and Declerck *et al.* 2003),
- the LP2 probe (SEQ ID n°23) described in Yamamoto *et al.* (Yamamoto *et al.* 1993),
- and all sequences having 70%, preferably 80% and more preferably 90% of identity with SEQ ID n°22 or SEQ ID n°23;
in this embodiment, the preferred helper probes for use in step (3) of the method are:
the HLEPGNE1L probe (SEQ ID n°24),
- the HLEPGNE1R probe (SEQ ID n°25),
- the HLP2L probe (SEQ ID n°26),
- the HLP2R probe (SEQ ID n°27),
- and all sequences having 70%, preferably 80% and more preferably 90% of identity with SEQ ID n°24, SEQ ID n°25, SEQ ID n°26 or SEQ ID n°27;
in this embodiment, the LEGPNE1 probe is preferably used in combination with HLEPGNE1L and HLEPGNE1R probes, and the LP2 probe is preferably used in combination with HLP2L and HLP2R probes.

| Probe | Sequence 5'-3' , | rRNA target position | Tm/%GC/n | Specificity |
|---|---|---|---|---|
| **LEGPNE1** | ATCTG ACCGT CCCAG GTT | 16S, 621-638 | 48/56/18 | *Legionella pneumophila, L walte* |
| **HLEGPNE1L** | AAGCC CAGGA ATTTC ACAGA TAACT TAATC | 16S, 620-591 | | |
| **HLEGPNE1R** | CCCTC TCCCA TACTC GAGTC AACCA GTATT | 16S, 639-668 | | |
| **LP2** | AGCTT TCATC CAAAG ATA | 16S, 183-200 | 39/33/18 | *Legionella pneumophila, L wadsw* |
| **HLP2L** | TTATG CGGTA TTAGC TTGAG TTTCC CCAAG | 16S, 182-153 | | |
| **HLP2R** | AATCT TAAAG CGCCA GGCCC GAAGG TCCCC | 16S, 201-230 | | |

Furthermore described herein is
the detection and enumeration of *Salmonella spp.* In this embodiment, the preferred specific labeled oligonucleotidic probes for use in step (2) of the method are:
- the Sal1 probe (SEQ ID n°28) described in Rönner and Stackebrandt (Rönner and Stackebrandt, 1994),
- the Sal3 probe (SEQ ID n°29) described in Nordentoft *et al.* and Oliveira and Bernardo (Nordentoft *et al.* 1997, Oliveira and Bernardo 2002),
- the Sal544 probe (SEQ ID n°30) described in Fang *et al.* (Fang *et al.* 2003),
- and all sequences having 70%, preferably 80% and more preferably 90% of identity with SEQ ID n°28, SEQ ID n°29, or SEQ ID n°30;
in this embodiment, the preferred helper probes for use in step (3) of the method are:
- the HSal1L probe (SEQ ID n°31),
- the HSal1R probe (SEQ ID n°32),
- the HSal3L probe (SEQ ID n°33),
- the HSal3R probe (SEQ ID n°34),
- the HSal544L probe (SEQ ID n°35),
- the HSal544R probe (SEQ ID n°36),
- and all sequences having 70%, preferably 80% and more preferably 90% of identity with SEQ ID n°31, SEQ ID n°32, SEQ ID n°33, SEQ ID n°34, SEQ ID n°35 or SEQ ID n°36;
in this embodiment, the Sall probe is preferably used in combination with HSal1L and HSal1R probes, the Sal3 probe is preferably used in combination with HSal3L and HSal3R probes, and the Sal544 probe is preferably used in combination with HSal544L and HSal544R probes.

| Probe | Sequence 5'-3' | rRNA target position | Tm/%GC/n | Specificity |
|---|---|---|---|---|
| **Sal1** | ACAGCA CATGC GCTTT TGTG | 23S, 341-360 | 55/50/20 | *Salmonella* spp |
| **Hsal1L** | TACGG GGCTG TCACC CTGTA TCGCG CGCCT | 23S, 311-340 | | |
| **Hsal1R** | ACCAC GTGTC CCGCC CTACT CATCG AGCTC | 23S, 361-391 | | |
| **Sal3** | AATCA CTTCA CCTAC GTG | 23S, 1713-1730 | 39/44/18 | *Salmonella* spp |
| **HSal3L** | TCAGC GTGCC TTCTC CCGAA GTTAC GGCAC | 23S,1683-1712 | | |
| **HSal3R** | TATCT TCGAC TGACT TCAGC TCCAT GAGTA | 23S, 1731-1760 | | |
| **Sal544** | GCAGT CACAC AGGTA AAC | 23S, 544-562 | 39/50/18 | *Salmonella* spp |
| **HSal544L** | CTGTG CTCCC ACTGC TTGTA CGTAC ACGGT | 23S, 514-543 | | |
| **Hsal544R** | TAAGT CGCTG ACCCCA TTATA CAAAA GGTAC | 23S, 563-592 | | |

The present invention is also directed to:
- helper probes for the detection and enumeration of *Escherichia coli* in a sample). Furthermore described herein are:
- helper probes for the detection and enumeration of *Legionella* spp. in a sample,
- helper probes for the detection and enumeration of *Legionella pneumophila* in a sample,
- helper probes for the detection and enumeration of *Salmonella* spp. in a sample, as described previously.

Helper probes are unlabeled oligonucleotides that bind to regions adjacent to that targeted by the specific labeled probe, which enhances *in situ* accessibility and hence the probe-conferred signal. Helper probes targeting the 5' and 3' adjacent regions of the specific probe are designed as follow.

A 30-nucleotides sequence, located at the 5' and 3' adjacent regions of the specific probe is identified by inspection of an alignment of targeted micro-organisms. The length of the helper sequence is dictated by the Tm (melting temperature), which must be close and preferably at least as high as, that of the specific probe. Specificity of the helper sequence designed is tested *in silico* by inspection of an alignment against a large rDNA sequences referenced database. The helper specificity must include the targeted micro-organism but not exclusively.

Hybridization conditions (buffer composition, such as salt, formamide concentrations, and temperature) optimised for the specific labeled probe are tested with the helpers. An evaluation of the positive effect of the unlabeled helpers when combined with the labeled specific probe is assessed by epifluorescent microscopy, flow cytometry or solid phase cytometry or with any equivalent detection instrument.

It falls within the ability of the skilled person knowing the present invention to design other helper probes specific to other microorganisms for the dectection and enumeration of these other microorganisms.

The present invention also provides a kit for detecting and enumerating viable *Escherichia coli* microorganisms in a sample suspected of containing said microorganisms comprising:
(1) means for contacting said sample with a cell nutritive resource and a cellular proliferation inhibitor,
(2) at least one fluorescence labeled oligonucleotidic probe able to specifically hybridize at least one portion of ribosomal nucleic acids of *Escherichia coli* microorganisms, said probe being chosen among ECOLI probe (SEQ ID n°1) or Colinsitu probe (SEQ ID n°2) and all sequences having 70%, preferably 80% and more preferably 90% of identity with SEQ ID n°1 or SEQ ID n°2,
(3) a least one helper probe able to hybridize at least one portion of said ribosomal nucleic acids of said *Escherichia coli* microorganism.

In a prefered embodiment, the present invention provides a kit as described above, wherein the helper probe is selected from the group consisting of the HECOLIL probe (SEQ ID n°3), the HECOLIR (SEQ ID n°4), the HColinsituL (SEQ ID n°5),the HColinsituR (SEQ ID n°6) and all sequences having 70%, preferably 80% and more preferably 90% of identity with SEQ ID n°3, SEQ ID n°4, SEQ ID n°5 or SEQ ID n°6.

Furthermore described is a kit for increasing the fluorescence signal of a labeled oligonucleotidic probe able to specifically hybridize at least one portion of ribosomal nucleic acids of *Legionella spp* in a sample, containing:
(1) at least one fluorescence labeled oligonucleotidic probe able to specifically hybridize at least one portion of ribosomal nucleic acids of said microorganism, selected from the group consisting of the LEG705 probe (SEQ ID n°7), the LEG226 probe (SEQ ID n°8), the Legall11 probe (SEQ ID n°9), the Legall22 probe (SEQ ID n°10), the Leg120v probe (SEQ ID n°11), and all sequences having 70%, preferably 80% and more preferably 90% of identity with SEQ ID n°7, SEQ ID n°8, SEQ ID n°9, SEQ ID n°10 or SEQ ID n°11, and
(2) a least one helper probe able to hybridize at least one portion of said ribosomal nucleic acids of said microorganism, selected from the group consisting of the HLEG705L probe (SEQ ID n°12), the HLEG705R probe (SEQ ID n°13), the HLEG226L probe (SEQ ID n°14), the HLEG226R probe (SEQ ID n°15), the HLegall11L probe (SEQ ID n°16), the HLegall11R probe (SEQ ID n°17), the HLegall22L probe (SEQ ID n°18), the HLegall22R probe (SEQ ID n°19), the HLeg120vL (SEQ ID n°20), the HLeg120vR (SEQ ID n°21) and all sequences having 70%, preferably 80% and more preferably 90% of identity with SEQ ID n°12, SEQ ID n°13, SEQ ID n°14, SEQ ID n°15, SEQ ID n°16, SEQ ID n°17, SEQ ID n°18, SEQ ID n°19, SEQ ID n°20 or SEQ ID n°21.

Furthermore described is a kit for increasing the fluorescence signal of a labeled oligonucleotidic probe able to specifically hybridize at least one portion of ribosomal nucleic acids of *Legionella pneumophila* in a sample, containing:
(1) at least one fluorescence labeled oligonucleotidic probe able to specifically hybridize at least one portion of ribosomal nucleic acids of said microorganism, selected from the group consisting of the LEGPNE1 probe (SEQ ID n°22), the LP2 probe (SEQ ID n°23), and all sequences having 70%, preferably 80% and more preferably 90% of identity with SEQ ID n°22 or SEQ ID n°23, and
(2) a least one helper probe able to hybridize at least one portion of said ribosomal nucleic acids of said microorganism, selected from the group consisting of the HLEPGNE1L probe (SEQ ID n°24),the HLEPGNE1R probe (SEQ ID n°25), the HLP2L probe (SEQ ID n°26),the HLP2R probe (SEQ ID n°27), and all sequences having 70%, preferably 80% and more preferably 90% of identity with SEQ ID n°24, SEQ ID n°25, SEQ ID n°26 or SEQ ID n°27.

Furthermore described is a kit for increasing the fluorescence signal of a labeled oligonucleotidic probe able to specifically hybridize at least one portion of ribosomal nucleic acids of *Salmonella* spp in a sample, containing:
(1) at least one fluorescence labeled oligonucleotidic probe able to specifically hybridize at least one portion of ribosomal nucleic acids of said microorganism, selected from the group consisting of the Sall probe (SEQ ID n°28), the Sa13 probe (SEQ ID n°29), the Sa1544 probe (SEQ ID n°30), and all sequences having 70%, preferably 80% and more preferably 90% of identity with SEQ ID n°28, SEQ ID n°29, or SEQ ID n°30, and
(2) a least one helper probe able to hybridize at least one portion of said ribosomal nucleic acids of said microorganism, selected from the group consisting of the HSal1L probe (SEQ ID n°31), the HSal1R probe (SEQ ID n°32), the HSal3L probe (SEQ ID n°33), the HSa13R probe (SEQ ID n°34), the HSa1544L probe (SEQ ID n°35), the HSa1544R probe (SEQ ID n°36), and all sequences having 70%, preferably 80% and more preferably 90% of identity with SEQ ID n°31, SEQ ID n°32, SEQ ID n°33, SEQ ID n°34, SEQ ID n°35 or SEQ ID n°36.

The present invention is illustrated in the following examples, which should not be interpreted in any way for narrowing the scope of this invention.

### EXAMPLES

### 1. Design of helper probes

Helper probes (30 nucleotides) were designed to the 5' and the 3' adjacent regions of the *E. coli* specific labeled probes (ECOLI and COLINSITU).

The ECOLI probe targets the 453-475 position of the *E. coli* 16SrDNA gene.

The helper probe sequence to the 5' adjacent region of the ECOLI probe is :
HECOLIL (5'-3') : TTCCT CCCCG CTGAA AGTAC TTTAC ACCCG and targets the 425-452 position of the *E. coli* 16SrDNA gene.

The helper probe sequence to the 3' adjacent region of the ECOLI probe is :
HECOLIR (5'-3') : CGGTG CTTCT TCTGC GGGTA ACGTC AATGA and targets the 476-505 position of the *E. coli* 16SrDNA gene.

The COLINSITU probe targets the 637-660 position of the *E. coli* 16SrDNA gene. The helper probe sequence to the 5' adjacent region of the COLINSITU probe is :
HCOLINSITUL : ATGCA GTTCC CAGGT TGAGC CCGGG GATTT and targets the 617-636 position of the *E. coli* 16SrDNA gene.

The helper probe sequence to the 3' adjacent region of the COLINSITU probe is :
HCOLINSITUR : CGCTA CACCT GGAAT TCTAC CCCCC TCTAC and targets the 661-684 position of the *E. coli* 16SrDNA gene.

Helpers sequences were first identified by inspection of an alignment of *E. coli* sequences referenced within 16S rDNA database (Genbank), using any phylogenic software, such as for example ARB software. The length of each helper probe was reduced from 30 nucleotides to 18 nucleotides for HECOLIL, 19 nucleotides for HECOLIR, 19 nucleotides for HCOLINSITUL and 24 nucleotides for HCOLINSITUR, to adjust the Tm to that of the specific probe.

Specificity of the helper was verified *in silico* by inspection of aligned *E. coli* sequences referenced from sequence 16S rDNA databases.

Specificity of the helpers targeted *E. coli* members but not be exclusive.

Hybridization conditions optimised for the specific labeled probe were tested with the helpers on several *E. coli* strains. The HECOLIR helpers did not enhance the fluorescence-conferred probes and was not retained.

The complete set of probes (included labeled specific oligonucleotides and unlabeled helpers) designed for the *E. coli* specific detection was evaluated on a large diversity of *E. coli* and non-*E.coli* strains to assess his specificity. The DNA sequences of the designed helpers are presented in Table 1.

**Table 1**

| **PROEE/HELPERS** | **PROBB SEQUBKCB (5' - 3')** | **rRNA TARGET POSITION*** | **SFECIFICITT** | **REFERENCES** |
|---|---|---|---|---|
| ECOLI (specific probe) | GCAAAGGTATTAACTTTACTCCC | 16S, 453-475 | *Escherichia coli* | McGregor et al., 1996 |
| HECOLIL | TTCCTCCCCGCTGAAAGT | 16S, 435-452 | *E. coli* | The present invention |
| Colinsitu (specific probe) | GAGACTCAAGATTGCCAGTATCAG | 16S, 637-660 | *Escherichia coli* and *Shigella* Shigella | Regnault et al., 2000 |
| HColinsituL | ATGCAGTTCCCAGGTTGAG | 16S, 617-636 | *E. coli* | The present invention |
| HColinsituR | ACCTGGAATTCTACCCCCCTCTAC | 16S, 661-684 | *E. coli* | The present invention |

### 2. Material and Method

### Probes.

The 16S rRNA probes specific of *E. coli,* ECOLI and Colinsitu, used in this example are described respectively in McGregor *et al.* and in Regnault et al. (McGregor *et al.* 1996, Regnault *et al.* 2000). The 5' fluorescein isothiocyanate (FITC)-ECOLI probe and the 5' FITC-Colinsitu probe were purshased from ThermoElectron, Ulm, Germany. Helper probes HECOLIL, HECOLIR, HColinsituL and HColinsituR were designated as described previously.

### Cell culture

Strains were cultured on TCS broth for 18-24 h at 37°C for *Enterobacteriaceae* strains and at 30°C for non-*Enterobacteriaceae* strains. To obtain cells in stationary growth phase (low rRNA content), 1% of the previous culture was inoculated into a fresh TSC broth and cultured for 18-24h at 37°C for *Enterobacteriaceae* strains and at 30°C for non-*Enterobacteriaceae* strains. Following the culture step, cells were suspended in PBS and cell suspensions were calibrated to 10²-10³cells/ml.

One hundred microliters of cell suspension on stationary growth phase were then filtered through a CB04 membrane.

### DVC procedure

One hundred microliters of cell suspension on stationary growth phase were filtered through a CB04 membrane and placed on labeling pads soaked in 550 µl of non-selective nutritive broth (70% PBS, 0.036% yeast extract, 0.36% casamino acid) containing nalidixic acid (final concentration 10 µg/ml, Sigma) in petri dishes. The samples were then incubated at 37°C for 4h.

### FISH protocol

Untreated cells and DVC treated cells were fixed on labeling pas soaked in 550 µl of 80% ethanol. Membranes and pad were incubated in petri dishes at room temperature for about 5 min then dried at romm temperature for 3 min. Membranes were placed in 50 µl of hybridization buffer (0.9 mM NaCl, 20 mM Tris-HCl pH7.2, 0.01% sodium dodecyl sulfate, 20% formamide dionized, 0.5% bovine serum albumine, 0.1 mg/ml Poly(A), 0.01% evans blue) comprising respective probes (final concentration of each oligonucleotidic FITC labeled and unlabeled probes was 2.5ng/µl) in petri dishes and incubated at 46°C for 1.5h. Following hybridization membranes were placed on labeling pad soaked in washing buffer (40 mM NaCl, 20 mM Tris-HCl pH 7.2, 0.01% sodium dodecyl sulfate, 5 mM EDTA, 0.01% evans blue) and incubated at room temperature until the ChemScanRDI analysis.

### ChemScan RDI analysis

Each stained membrane was placed onto the sample holder of the ChemScanRDI on top of a 25-mm, 0.45 µm pore size black cellulose membrane (support pad, Chemunex) satured by 80 µl of washing buffer. The ChemScanRDI system consisted to a scan of the CB04 membrane with a beam argon laser (488 nm emission wavelenght). All fluorescent events emitting at 500-530 nm and 540-570 nm were collected by two photomultipliers and converted in numerized signals to the ChemScanRDI software (Chemunex). Fluorescent signal were discriminated from raw data by use of internal discriminants to differentiate between stained cells and autofluorescent particles. The aim discriminants applied on each fluorescent event were: Peak Intensity, Secondary/Primary ratio according 540-570 nm emission channel and 500-530 nm emission channel respectively, Number of lines (nL) and Number of samples (nS). At the end of the analysis procedure, results were plotted on a schematic membrane (map) on which all discriminated fluorescent events were positionned (x and y coordinates). The entire scan of the 25 mm membrane and discriminate processes were performed in 3 min. A final validation step of discriminated fluorescent events, by epifluorescent microscopic observation is required and made as possible by using a BX60 epifluorescent microscope with a WIBA filter fitted on motorized stage driven by the SPC. In this example, three discriminants parameters determined with the ScanRDI software were used to characterize positive hybridized cells: Peak intensity fluorescence (PIF) of the dominant peak expressed in arbitrary fluorescents units (FU), and numbers of scan lines (nL) and scan samples (nS), which were both linked to the fluorescence intensity and the cellular lenght in the x axis and the y axis respectively. The value of nL x nS product is useful to give a cell size index. Consequently, PIF and the product nL x nS were considered to be respectively an expression of the intracellular content of 16SrRNA targets and an expression of the rate of cell elongation.

### 3. Results

Untreated DVC cells and DVC treated cells (*E. coli* ATCC 11775) were hybridized with different probes combinations comprising or not helper probes. The fluorescent signals of hybridized cells were assessed by the ChemScanRDI and the results are summerized in Table 2.

**Table 2**

| Probe | ScanRDI counts | | Fluorescence |
|---|---|---|---|
| | Untreated DVC | DVC treated | |
| combination | cells | cells | intensity (FU) |
| COLINSITU-FITC | 0 | 0 | 0 |
| ECOLI-FITC | 0 | 4.5 (6.4) | 118.0 (19.4) |
| COLINSITU-FITC | 0 | 46.5 (19.1) | 142.6 (14.6) |
| + ECOLI-FITC | | | |
| COLINSITU-FITC | | | |
| + ECOLI-FITC + | 0 | 152.5 (75.7) | 660.4 (206.4) |
| helper probes | | | |

None *E. coli* hybridized cells untreated by the DVC procedure was detected by the ChemScanRDI. The ribosomal content of untreated DVC cells is too low and generates weak fluorescent signal under the detection threshold of the ChemScanRDI however the FISH protocol used. Comparatively, DVC treated cells were detected by the ChemScanRDI, excepted for cells hybridized with the FITC-Colinsitu probe. The use of two FITC-labeled probes allows a weak amplification (x 1.2) of the florescence intensity of hybridized cells comparatively to a single FITC-labeled probe, although the use of helper probes in combination with FITC-labeled probes allows a stronger amplification (x 5.6) of the fluorescence intensity of hybridized cells.

The specificity of the helper probes designed for the *E. coli* detection was tested on 74 *eubacteria* strains including *E. coli* (n=29), two enteropathogenic *E. coli* strains (serotypes O157:H7 and O126:B16), other members of *Enterobacteriaceae* family (n=33), and non-*Enterobacteriaceae* strains (n=12) (Table 3). Assays were performed using FITC-Colinsitu and FITC-ECOLI probes and the four designed helper probes on midlog growth phase cells. All *E. coli* strains tested gave a positive hybridization signal including the two enteropathogenic strains. The probes did not cross-react with other members of *Enterobacteriaceae,* or with non-*Enterobacteriaceae* strains excepted *Shigella boydii, S. dysenteria and S. sonnei.* This cross-reaction was already reported by Regnault et al. (Regnault et al. 2000), who disclosed a 100% homology of rrs sequences from the Colinsitu target sequence (position 637-660) between *E. coli, E. fergusonii, Shigella flexneri, Shigella dysenteriae, Shigella sonnei* and *Shigella boydii.* However, in the present example, fluorescence intensity of hybridized *Shigella* strains cells was weak comparatively to all hybridized *E. coli* strains, which showed a strong fluorescent signal. The cross-reaction detected for *Shigella spp.* was already reported by Regnault *et al.* (2000) which reported a 100% of *rrs* sequences homology from the Colinsitu target sequence (position 637-660) for *E. coli, E. fergusonii, Shigella flexneri, Shigella dysenteriae, Shigella sonnei* and *Shigella boydii.* Similarly, we found the same matching for *E. coli, E. albertii, Shigella flexneri, Shigella dysenteriae, Shigella sonnei* and *Shigella boydii* when ECOLI target sequence is submitted to GenBank.

The present invention showed a detection limit to one single *E. coli* viable cells per filtered volume sample. The analyzed volume is determined as the maximum volume of water sample allowing a ChemScanRDI detection. For example, it was defined as 500mL for tap water and bottled water, and 25mL for seawater. The present invention allowed the dectection of a single *E. coli* cell among 10⁷-10⁸ non targeted cells according the water sample (for example, the detection limit in marine recreational water, was one cell among 10⁷ non targeted cells).

**Table 3 :Origins and phylogenetic affiliations of bacterial strains used in this study**

| Organim and phylogenetic | Source | Taxonomic familly |
|---|---|---|
| affiliation | | |
| **Eubacteria** | | |
| **Actinobacteria** | | |
| *Micrococcus luteus* | ATCC 9341 | *Microccoceae* |
| **Firmicute** | | |
| *Lactobacillus lactis lactis* | CIP 70.57 | *Lactobacillaceae* |
| *Staphylococcus aureus* | ATCC 6538 | *Staphylococcaceae* |
| *Bacillus subtilis* | ATCC 6653 | *Bacillaceae* |
| *Enterococcus faecalis* | CIP 106877 | *Enterococcaceae* |
| *Enterococcus faecium* | ATCC10541 | *Enterococcaceae* |
| **Proteobacteria** | | |
| α**-subgroup** | | |
| *Pseudomonas diminuta* | ATCC 11569 | *Caulobacteriaceae* |
| γ**-subgroup** | | |
| *Pseudomonas aeruginosa* | ATCC 19429 | *Pseudomonaceae* |
| *Pseudomonas aeruginosa* | ATCC 27853 | *Pseudomonaceae* |
| *Pseudomonas aeruginosa* | ATCC 15442 | *Pseudomonaceae* |
| *Pseudomonas putida* | OOB | *Pseudomonaceae* |
| *Pseudomonas syringae* | OOB | *Pseudomonaceae* |
| *Citrobacter freundii* | OOB | *Enterobacteriaceae* |
| *Escherichia coli* | ATCC 11775 | *Enterobacteriaceae* |
| *Escherichia coli* | ATCC 8739 | *Enterobacteriaceae* |
| *Escherichia coli* | ATCC 104130 | *Enterobacteriaceae* |
| *Escherichia coli 0157:H.7* | ATCC 35150 | *Enterobacteriaceae* |
| *Escherichia coli 0126:Bl6* | OOB | *Enterobacteriaceae* |
| *Escherichia coli K12* | OOB | *Enterobacteriaceae* |
| *Escherichia coli (n = 23)* | OOB | *Enterobacteriaceae* |
| *Enterobacter aerogenes* | ATCC 13048 | *Enterobacteriaceae* |
| *Enterobacter cloacae (n =3)* | OOB | *Enterobacteriaceae* |
| *Enterobacter sakazaki* | OOB | *Enterobacteriaceae* |
| *Enterobacter gergoviae* | ATCC33028 | *Enterobacteriaceae* |
| *Klebsiella ornithinolytica* | CIP 103334T | *Enterobacteria ceae* |
| *Klebsiella oxytoca* | ATCC 13182 | *Enterobacteriaceae* |
| *Klebsiella oxytoca* | CIP 7932 | *Enterobacteriaceae* |
| *Raoultella planticola* | ATCC 33531 | *Enterobacteriaceae* |
| *Raoultella planticola* | ATCC 33558 | *Enterobacteriaceae* |
| *Klebsiella pneumoniae* | ATCC 13883 | *Enterobacteriaceae* |
| *pneumoniae* | | |
| *Klebsiella pneumoniae* | OOB | *Enterobacteriaceae* |
| *pneumoniae (n* = 3) | | |
| *Klebsiella pneumoniae* | ATCC 13884 | *Enterobacteriaceae* |
| *rhinoscleromatis* | | |
| *Raoultella terrigena* | ATCC 133257 | *Enterobacteriaceae* |
| *Klebsiella spp.* | OOB | *Enterobacteriaceae* |
| *Proteus mirabilis* | ATCC 10005 | *Enterobacteriaceae* |
| *Salmonella typhimurium LT2* | ATCC 43971 | *Enterobacteriaceae* |
| *Salmonella typhimurium* | CIP 103446 | *Enterobacteriaceae* |
| *Salmonella typhimurium* | OOB | *Enterobacteriaceae* |
| *Salmonella* indiana (n=2) | OOB | *Enterobacteriaceae* |
| *Salmonella* enteritidis | OOB | *Enterobacteriaceae* |
| *Salmonella parayphi B* | OOB | *Enterobacteriaceae* |
| *Salmonella virchow* | OOB | *Enterobacteriaceae* |
| *Serratia marcescens* | CIP58.64 | *Enterobacteriaceae* |
| *Shigella boydii* | OOB | *Enterobacteriaceae* |
| *Shigella dysenteriae* | OOB | *Enterobacteriaceae* |
| *Shigella sonnei* | OOB | *Enterobacteriaceae* |
| *Shigella fexneri* | CIP 82.48T | *Enterobacteriaceae* |

| | | |
|---|---|---|
| ATCC : American Type Culture Collection. CIP : Collection Institut Pasteur-France OOB : Culture collection maintened by the Oceanological Observatory of Banyuls sur mer - France | | |

### References

1. Amann R.I. Ludwig W. and Schleifer K.H. 1995. Phylogenetic identification and in situ detection of individual microbial cells without cultivation. Microbiol. Rev. 59 : 143-169.
2. Baudart, J., Coallier J., Laurent P and Prévost M. 2002. Rapid and sensitive enumeration of viable diluted cells of members of the family Enterobacteriaceae cells in freshwater and drinking waters. Appl. Environ. Microbiol. 68:5057-5063..
3. Bej A.K., Mahbubani M.H., and Atlas R.M. 1991. Detection of viable Legionella pneumophila in water by polymerase chain reaction and gene probes methods. Appl. Environ. Microbiol. 57 :597-600.
4. Buchbinder S, Trebesius K, Heesemann J. 2002. Evaluation of detection of Legionella spp. in water samples by fluorescence in situ hybridization, PCR amplification and bacterial culture. Int J Med Microbiol. 292:241-5.
5. Declerck P., Verelst L., Duvivier L., Van Damme A., Ollevier F. 2003. A detection method for Legionella spp in (cooling) water : fluorescent in situ hybridization (FISH) on whole bacteria. Wat Sci. Technol. 47 : 143-146.
6. Devos L., Clymans K., Boon N., and Verstraete W. 2005. Evaluation of nested PCR assays for the detection of Legionella pneumophila in a wide range of aquatic samples. J. App. Microbiol. 99 : 916-925.
7. Fang Q., Brockmann S., Botzenhart K., and Wiedenmann A. 2003. Improved detection of Salmonella spp., in foods by fluorescent in situ hybridization with 23sRNA probes : a comparison with conventional culture methods. J. Food Protection. 66 : 723-731.
8. Grimm D., Merkert H., Ludwig W., Schleifer K-H., Hacker J., and Brand B.C. 1998. Specific detection of Legionella pneumophila: construction of a new 16S rRNA-targeted oligonucleotide probe. Appl. Environ. Microbiol. 64: 2686-2690.
9. Kogure, K., Simidu U., Taga N., and Colwell R.R. 1987. Correlation of direct viable counts with heterotrophic activity for marine bacteria. Appl. Environ. Microbiol. 53 : 2332-2337.
10. Mc Gregor D.P., Fortser S., Steven J., Adair J., Leary S. E. C., Leslie D. L., Harris W.J. and Titball R.W. 1996. Simultaneous detection of microorganisms in soil suspension based on PCR amplification of bacterial 16S rRNA fragments.Biotechniques.21 : 463-471.
11. Leskela T, Tilsala-Timisjarvi A, Kusnetsov J, Neubauer P, Breitenstein A. 2005. Sensitive genus-specific detection of Legionella by a 16S rRNA based sandwich hybridization assay. J Microbiol Methods. 62:167-79.
12. Manz W., Amann R., Szewzyk R., Szewzyk U., Stenstrôm T.-A., Hutzler P., and Schleifer K.-H. 1995. In situ identification of Legionellaceae using 16S rRNA-targeted oligonucleotide probes and confocal laser scanning microscopy. Microbiology. 141 : 29-39.
13. Nordentoft S., Christensen H., and Wegener H.C. 1997. Evaluation of a fluorescent-labeled oligonucleotide probe targeting 23S rRNA for in situ detection of Salmonella serovars in paraffin-embedded tissue sections and their rapid identification in bacterial smears. J. Clin. Microbiol. 35 : 2642-2648.
14. Oliveira M., and Bernardo F. 2002. Fluorescent In situ hybridization applied to the rapid detection of Salmonella isolates from food and environmental origins. Revista Portuguesa de Ciências Veterinárias. 97 :81-85.
15. Regnault B., Martin-Delautre S., Lejay-Collin M., Lefèvre M., Grimont P.A.D. 2000. Oligonucleotide probe for the visualization of Escherichia coli/Escherichia fergusonii cells by in situ hybridization: specificity and potential applications. Res Microbiol. 151:521-533.
16. Rönner and Stackebrandt 1994. Development of 23S rRNA oligonucleotide probes for the identification of Salmonella species. Syst. Appl. Microbiol. 17:257-264.
17. Yamamoto H., Hashimoto Y., and Ezaki T. 1993. Comparison of detection methods for Legionella species in enviornmental water by colony isolation, fluorescent antibody staining and polymerase chian reaction. Microbiol. Immun. 37:617-622.
18. Yanez M.A., Carrasco-Serrano C., Barbera V.M., Catalan V. 2005. Quantitative detection of Legionella pneumophila in water samples by immunomagnetic purification and real-time PCR amplification of the dotA gene. Appl. Environ. Microbiol. 71 : 3433-3441.

## Claims

1. A method for detecting and enumerating viable *Escherichia coli* microorganisms in a sample suspected of containing said microorganisms comprising:
(1) contacting said sample with a cell nutritive resource and a cellular proliferation inhibitor,
(2) contacting said sample with at least one fluorescence labeled oligonucleotidic probe able to specifically hybridize at least one portion of ribosomal nucleic acids of said *Escherichia coli* microorganisms, said probe being chosen among ECOLI probe (SEQ ID n°1) or Colinsitu probe (SEQ ID n°2) and all sequences having 70%, preferably 80% and more preferably 90% of identity with SEQ ID n°1 or SEQ ID n°2, thereby performing hybridization,
(3) contacting said sample with a least one helper probe able to hybridize at least one portion of said ribosomal nucleic acids of said *Escherichia coli* microorganisms,
(4) detecting and quantifying the fluorescent signal of said fluorescence labeled oligonucleotide probe, thereby detecting and enumerating the number of viable *Escherichia coli* microorganisms in said sample.

2. The method of claim 1, **characterized in that** the fluorescent signal is detected and quantified by a solid phase cytometer.

3. The method of claims 1 or 2, **characterized in that** in step (2), the sample is contacted with ECOLI and Colinsitu.

4. The method according to any one of claims 1 to 3, wherein the helper probe used in step (3) is selected from the group consisting of the HECOLIL probe (SEQ ID n°3), the HECOLIR (SEQ ID n°4), the HColinsituL (SEQ ID n°5), the HColinsituR (SEQ ID n°6) and all sequences having 70%, preferably 80% and more preferably 90% of identity with SEQ ID n°3, SEQ ID n°4, SEQ ID n°5 or SEQ ID n°6.

5. The method according to any one of claims 1 to 4, wherein step (2) and step (3) are carried out simultaneously.

6. Kit for detecting and enumerating viable *Escherichia coli* microorganisms in a sample suspected of containing said microorganisms comprising:
(1) means for contacting said sample with a cell nutritive resource and a cellular proliferation inhibitor,
(2) at least one fluorescence labeled oligonucleotidic probe able to specifically hybridize at least one portion of ribosomal nucleic acids of *Escherichia coli* microorganisms, said probe being chosen among ECOLI probe (SEQ ID n°1) or Colinsitu probe (SEQ ID n°2) and all sequences having 70%, preferably 80% and more preferably 90% of identity with SEQ ID n°1 or SEQ ID n°2,
(3) a least one helper probe able to hybridize at least one portion of said ribosomal nucleic acids of said *Escherichia coli* microorganism.

7. The kit according to claim 6 for detecting and enumerating *Escherichia coli* in a sample, wherein the helper probe is selected from the group consisting of the HECOLIL probe (SEQ ID n°3), the HECOLIR (SEQ ID n°4), the HColinsituL (SEQ ID n°5), the HColinsituR (SEQ ID n°6) and all sequences having 70%, preferably 80% and more preferably 90% of identity with SEQ ID n°3, SEQ ID n°4, SEQ ID n°5 or SEQ ID n°6.

8. Helper probes for the detection and enumeration of *Escherichia coli,* selected from the group consisting of HECOLIL probe (SEQ ID n°3), the HECOLIR (SEQ ID n°4), the HColinsituL (SEQ ID n°5), the HColinsituR (SEQ ID n°6) and all sequences having 70%, preferably 80% and more preferably 90% of identity with SEQ ID n°3, SEQ ID n°4, SEQ ID n°5 or SEQ ID n°6.

## Patentansprüche

1. Verfahren zum Nachweisen und Auszählen von lebensfähigen *Escherichia coli-*Mikroorganismen in einer Probe, von welcher vermutet wird, dass sie die Mikroorganismen enthält, umfassend:
(1) das Inkontaktbringen der Probe mit einer Zellernährungsressource und einem Zellproliferationsinhibitor,
(2) das Inkontaktbringen der Probe mit mindestens einer Fluoreszenz-markierten Oligonukleotid-Sonde, welche zum spezifischen Hybridisieren von mindestens einem Teil von ribosomalen Nukleinsäuren der *Escherichia coli-*Mikroorganismen in der Lage ist, wobei die Sonde ausgewählt ist unter ECOLI-Sonde (SEQ ID Nr. 1) oder Colinsitu-Sonde (SEQ ID Nr. 2) und allen Sequenzen mit 70 %, bevorzugt 80 % und stärker bevorzugt 90 % Identität mit SEQ ID Nr. 1 oder SEQ ID Nr. 2, wobei Hybridisierung durchgeführt wird,
(3) das Inkontaktbringen der Probe mit mindestens einer Helfersonde, welche zum Hybridisieren von mindestens einem Teil der ribosomalen Nukleinsäuren der *Escherichia* coli-Mikroorganismen in der Lage ist,
(4) das Nachweisen und Quantifizieren des Fluoreszenzsignals der Fluoreszenz-markierten Oligonukleotid-Sonde, wobei die Anzahl von lebensfähigen *Escherichia coli-*Mikroorganismen in der Probe nachgewiesen und ausgezählt wird.

2. Verfahren nach Anspruch 1, **dadurch gekennzeichnet, dass** das Fluoreszenzsignal durch ein Festphasenzytometer nachgewiesen und quantifiziert wird.

3. Verfahren nach den Ansprüchen 1 oder 2, **dadurch gekennzeichnet, dass** in Schritt (2) die Probe mit ECOLI und Colinsitu in Kontakt gebracht wird.

4. Verfahren gemäß einem der Ansprüche 1 bis 3, wobei die in Schritt (3) verwendete Helfersonde aus der Gruppe ausgewählt ist, welche aus der HECOLIL,-Sonde (SEQ ID Nr. 3), der HECOLIR (SEQ ID Nr. 4), der HColinsituL (SEQ ID Nr. 5), der HColinsituR (SEQ ID Nr. 6) und allen Sequenzen mit 70 %, bevorzugt 80 % und stärker bevorzugt 90 % Identität mit SEQ ID Nr. 3, SEQ ID Nr. 4, SEQ ID Nr. 5 oder SEQ ID Nr. 6 besteht.

5. Verfahren gemäß einem der Ansprüche 1 bis 4, wobei Schritt (2) und Schritt (3) gleichzeitig durchgeführt werden.

6. Kit zum Nachweisen und Auszählen von lebensfähigen *Escherichia coli*-Mikroorganismen in einer Probe, von welcher vermutet wird, dass sie die Mikroorganismen enthält, umfassend:
(1) Mittel zum Inkontaktbringen der Probe mit einer Zellernährungsressource und einem Zellproliferationsinhibitor,
(2) mindestens eine Fluoreszenz-markierte Oligonukleotidsonde, welche zum spezifischen Hybridisieren von mindestens einem Teil von ribosomalen Nukleinsäuren von *Escherichia coli*-Mikroorganismen in der Lage ist, wobei die Sonde ausgewählt ist unter ECOLI-Sonde (SEQ ID Nr. 1) oder Colinsitu-Sonde (SEQ ID Nr. 2) und allen Sequenzen mit 70 %, bevorzugt 80 % und stärker bevorzugt 90 % Identität mit SEQ ID Nr. 1 oder SEQ ID Nr. 2,
(3) mindestens eine Helfersonde, welche zum Hybridisieren von mindestens einem Teil der ribosomalen Nukleinsäuren des *Escherichia coli-*Mikroorganismus in der Lage ist.

7. Kit gemäß Anspruch 6 zum Nachweisen und Auszählen von *Escherichia coli* in einer Probe, wobei die Helfersonde aus der Gruppe ausgewählt ist, welche aus der HECOLIL-Sonde (SEQ ID Nr. 3), der HECOLIR (SEQ ID Nr. 4), der HColinsituL (SEQ ID Nr. 5), der HColinsituR (SEQ ID Nr. 6) und allen Sequenzen mit 70 %, bevorzugt 80 % und stärker bevorzugt 90 % Identität mit SEQ ID Nr. 3, SEQ ID Nr. 4, SEQ ID Nr. 5 oder SEQ ID Nr. 6 besteht.

8. Helfersonden zum Nachweisen und Auszählen von *Escherichia coli,* welche aus der Gruppe ausgewählt sind, die aus HECOLIL-Sonde (SEQ ID Nr. 3), der HECOLIR (SEQ ID Nr. 4), der HColinsituL (SEQ ID Nr. 5), der HColinsituR (SEQ ID Nr. 6) und allen Sequenzen mit 70 %, bevorzugt 80 % und stärker bevorzugt 90 % Identität mit SEQ ID Nr. 3, SEQ ID Nr. 4, SEQ ID Nr. 5 oder SEQ ID Nr. 6 besteht.

## Revendications

1. Procédé de détection et d'énumération de micro-organismes *Escherichia coli* viables dans un échantillon suspecté de contenir lesdits micro-organismes comprenant :
(1) la mise en contact dudit échantillon avec une ressource nutritive cellulaire et un inhibiteur de prolifération cellulaire,
(2) la mise en contact dudit échantillon avec au moins une sonde d'oligonucléotide marquée par fluorescence, capable de s'hybrider spécifiquement à au moins une partie d'acides nucléiques ribosomaux desdits micro-organismes *Escherichia coli,* ladite sonde étant choisie parmi une sonde ECOLI (SEQ ID N° : 1) ou une sonde Colinsitu (SEQ ID N° : 2) et toutes les séquences ayant 70 %, de préférence 80 % et de manière davantage préférée, 90 % d'identité avec SEQ ID N° 1 ou SEQ ID N° 2, réalisant ainsi l'hybridation,
(3) la mise en contact dudit échantillon avec au moins une sonde auxiliaire capable de s'hybrider à au moins une partie desdits acides nucléiques ribosomiques desdits micro-organismes *Escherichia coli,*
(4) la détection et la quantification du signal fluorescent de ladite sonde d'oligonucléotide marquée par fluorescence, détectant ainsi et énumérant le nombre de micro-organismes *Escherichia coli* viables dans ledit échantillon.

2. Procédé selon la revendication 1, **caractérisé en ce que** le signal fluorescent est détecté et quantifié par un cytomètre en phase solide.

3. Procédé selon les revendications 1 ou 2, **caractérisé en ce que**, à l'étape (2), l'échantillon est mis en contact avec ECOLI et Colinsitu.

4. Procédé selon l'une quelconque des revendications 1 à 3, dans lequel la sonde auxiliaire utilisée à l'étape (3) est choisie dans le groupe comprenant la sonde HECOLIL, (SEQ ID N° : 3), HECOLIR (SEQ ID N° : 4), HColinsitu (SEQ ID N° : 5), HColinsituR (SEQ ID N° : 6) et toutes les séquences ayant 70 %, de préférence 80 % et de manière particulièrement préférée, 90 % d'identité avec SEQ ID N° : 3, SEQ ID N° : 4, SEQ ID N° : 5 ou SEQ ID N° : 6.

5. Procédé selon l'une quelconque des revendications 1 à 4, dans lequel l'étape (2) et l'étape (3) sont réalisées simultanément.

6. Kit de détection et d'énumération de micro-organismes *Escherichia coli* viables dans un échantillon suspecté de contenir lesdits micro-organismes comprenant :
(1) des moyens de mise en contact dudit échantillon avec une ressource nutritive cellulaire et un inhibiteur de prolifération cellulaire,
(2) au moins une sonde d'oligonucléotide marquée par fluorescence, capable de s'hybrider spécifiquement à au moins une partie d'acides nucléiques ribosomaux desdits micro-organismes *Escherichia coli,* ladite sonde étant choisie parmi une sonde ECOLI (SEQ ID N° : 1) ou une sonde Colinsitu (SEQ ID N° : 2) et toutes les séquences ayant 70 %, de préférence 80 % et de manière davantage préférée, 90 % d'identité avec SEQ ID N° 1 ou SEQ ID N° 2,
(3) au moins une sonde auxiliaire capable de s'hybrider à au moins une partie desdits acides nucléiques ribosomiques desdits micro-organismes *Escherichia coli.*

7. Kit selon la revendication 6, pour détecter et énumérer *Escherichia coli* dans un échantillon, dans lequel la sonde auxiliaire est choisie dans le groupe comprenant la sonde HECOLIL, (SEQ ID N° : 3), HECOLIR (SEQ ID N° : 4), HColinsitu (SEQ ID N° : 5), HColinsituR (SEQ ID N° : 6) et toutes les séquences ayant 70 %, de préférence 80 % et de manière particulièrement préférée, 90 % d'identité avec SEQ ID N° : 3, SEQ ID N° : 4, SEQ ID N° : 5 ou SEQ ID N° : 6.

8. Sondes auxiliaires pour la détection et l'énumération de *Escherichia coli,* choisies dans le groupe comprenant la sonde HECOLIL (SEQ ID N° : 3), HECOLIR (SEQ ID N° : 4), HColinsitu (SEQ ID N° : 5), HColinsituR (SEQ ID N° : 6) et toutes les séquences ayant 70 %, de préférence 80 % et de manière particulièrement préférée, 90 % d'identité avec SEQ ID N° : 3, SEQ ID N° : 4, SEQ ID N° : 5 ou SEQ ID N° : 6.
